(19)

Europäisches
Patentamt

European
Patent Office

Office européen
des brevets

(11)  **EP 4 521 098 A1**

(12)  # EUROPEAN PATENT APPLICATION
published in accordance with Art. 153(4) EPC

(43) Date of publication:
**12.03.2025 Bulletin 2025/11**

(21) Application number: **23815096.5**

(22) Date of filing: **26.05.2023**

(51) International Patent Classification (IPC):
***G01N 21/31*** (2006.01)    ***G01N 21/25*** (2006.01)

(52) Cooperative Patent Classification (CPC):
**A61B 5/00; A61B 5/02; G01N 21/15; G01N 21/25; G01N 21/31; G02B 5/28**

(86) International application number:
**PCT/CN2023/096493**

(87) International publication number:
**WO 2023/231913 (07.12.2023 Gazette 2023/49)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **02.06.2022 CN 202210622250**

(71) Applicant: **Huawei Technologies Co., Ltd.
Shenzhen, Guangdong 518129 (CN)**

(72) Inventors:
• **YANG, Hui
Shenzhen, Guangdong 518129 (CN)**

• **YANG, Sulin
Shenzhen, Guangdong 518129 (CN)**
• **JIANG, Hongyan
Tianjin 300308 (CN)**
• **ZHANG, Chen
Tianjin 300308 (CN)**
• **LIU, Shuyang
Tianjin 300308 (CN)**
• **LIU, Chang
Shenzhen, Guangdong 518129 (CN)**

(74) Representative: **Pfenning, Meinig & Partner mbB
Patent- und Rechtsanwälte
Theresienhöhe 11a
80339 München (DE)**

(54)  ## OPTICAL MEASUREMENT APPARATUS, BANDPASS FILTER, OPTICAL MEASUREMENT METHOD, AND ELECTRONIC DEVICE

(57)    An optical measurement apparatus (100), a bandpass filter (20), an optical measurement method, and an electronic device are provided. The optical measurement apparatus (100) includes a light source emitting assembly (10), a bandpass filter (20), and a receiving and measuring assembly (30). The bandpass filter (20) is disposed between a to-be-measured object (40) and the receiving and measuring assembly (30). An optical filter spectrum band of the bandpass filter (20) includes a light source operating spectrum band of the light source emitting assembly (10). The light source emitting assembly (10) is configured to emit first detection light to the to-be-measured object (40). The bandpass filter (20) is configured to emit third detection light to the receiving and measuring assembly (30) based on second detection light. The second detection light includes light reflected or transmitted by the to-be-measured object (40) based on the first detection light. The receiving and measuring assembly (30) is configured to determine a to-be-measured feature value of the to-be-measured object (40) based on the first detection light and the third detection light. The optical measurement apparatus (100) can simply and effectively remove interference optical signals through the bandpass filter (20), and has high measurement precision and high practicability.

FIG. 1

EP 4 521 098 A1

**EP 4 521 098 A1**

## Description

This application claims priority to Chinese Patent Application No. 202210622250.3, filed with the China National Intellectual Property Administration on June 02, 2022 and entitled "OPTICAL MEASUREMENT APPARATUS, BANDPASS FILTER, OPTICAL MEASUREMENT METHOD, AND ELECTRONIC DEVICE", which is incorporated herein by reference in its entirety.

## TECHNICAL FIELD

This application relates to the field of optical technologies, and in particular, to an optical measurement apparatus, a bandpass filter, an optical measurement method, and an electronic device.

## BACKGROUND

With deepening of optical research, optical measurement apparatuses based on optical measurement technologies are gradually proposed, for example, a pulse wave sensing apparatus and a three-dimensional optical measurement apparatus of a human body outline that are commonly used in the biomedical field and that are implemented by using a photoplethysmography (photoplethysmography, PPG for short) technology. With wide application of these optical measurement apparatuses, people raise higher requirements on measurement accuracy of these optical measurement apparatuses.

An interference signal (for example, an external interference signal like external ambient light and an internal noise of an optical measurement apparatus) in an actual measurement process greatly affects signal-to-noise ratio performance of the optical measurement apparatus. Consequently, measurement accuracy of the optical measurement apparatus is reduced. In the conventional technology, digital signal processing is usually performed, according to various built-in filter algorithms of the optical measurement apparatus, on a signal obtained through measurement by the optical measurement apparatus, to remove the interference signal. However, this imposes a higher requirement on a data processing capability of the optical measurement apparatus; and these filter algorithms are easily affected by performance such as a sampling frequency of the optical measurement apparatus, and it is difficult to completely filter out the interference signal. As a result, the existing optical measurement apparatus that filters out the interference signal according to the filter algorithm needs to have a high data processing capability, measurement accuracy of the optical measurement apparatus is not ideal, and practicability is poor.

## SUMMARY

To resolve the foregoing problems, this application provides an optical measurement apparatus, a bandpass filter, an optical measurement method, and an electronic device. The optical measurement apparatus may reduce or even eliminate interference light received by the optical measurement apparatus through the bandpass filter radically. In this way, measurement accuracy of the optical measurement apparatus is high, and practicability is high.

According to a first aspect, an embodiment of this application provides an optical measurement apparatus. The optical measurement apparatus includes a light source emitting assembly, a bandpass filter, and a receiving and measuring assembly. The bandpass filter is disposed between a to-be-measured object and the receiving and measuring assembly. An optical filter spectrum band of the bandpass filter includes a light source operating spectrum band of the light source emitting assembly. In actual operation, the light source emitting assembly is configured to emit first detection light to the to-be-measured object. The bandpass filter is configured to emit third detection light to the receiving and measuring assembly based on second detection light. Herein, the second detection light includes light reflected or transmitted by the to-be-measured object based on the first detection light. The receiving and measuring assembly is configured to determine a to-be-measured feature value of the to-be-measured object based on the first detection light and the third detection light.

In the foregoing implementation, one bandpass filter whose optical filter spectrum band includes the light source operating spectrum band of the light source emitting assembly is disposed between the receiving and measuring assembly of the optical measurement apparatus and the to-be-measured object. Interference light emitted to the receiving and measuring assembly is filtered out through the bandpass filter, so that the receiving and measuring assembly receives only valid signal light that is sent by the light source emitting assembly and that is reflected or transmitted by the to-be-measured object, and the interference light is eliminated radically. This manner is simple and effective, and can significantly improve measurement accuracy and practicability of the optical measurement apparatus.

With reference to the first aspect, in a feasible implementation, the bandpass filter includes a substrate and a thin-film optical filter disposed on the substrate. A film structure of the thin-film optical filter includes at least two Fabry-Perot cavities disposed in series. Structures of all of the at least two Fabry-Perot cavities are the same. A first Fabry-Perot cavity and a second Fabry-Perot cavity, which are any two adjacent cavities of the at least two Fabry-Perot cavities, are

2

connected through a connection layer. A side that is of the connection layer and that is connected to the first Fabry-Perot cavity and a side that is of the connection layer and that is connected to the second Fabry-Perot cavity each are a low refractive index layer of one basic thickness. A film structure of the first Fabry-Perot cavity is $(xHyL)^n kH(yLxH)^n$. Herein, H represents a high refractive index layer of one basic thickness, L represents a low refractive index layer of one basic thickness, x is an optical thickness coefficient of the high refractive index layer, y is an optical thickness coefficient of the low refractive index layer, xH represents a high refractive index layer of x basic thicknesses, yL represents a low refractive index layer of y basic thicknesses, kH represents a high refractive index material layer of k basic thicknesses, n is a quantity of periodicities of a first membrane stack xHyL and a second membrane stack yLxH, and k is a multiple of 2. The basic thickness is an optical thickness of a quarter of a center wavelength.

In the foregoing implementation, the bandpass filter is implemented by disposing Fabry-Perot cavities in series. In this way, a bandpass gradient of the bandpass filter can be increased, and a rectangularity of a passband curve is more obvious, so as to ensure that a width and a transmittance of the optical filter spectrum band of the bandpass filter and a cut-off rate of the bandpass filter can meet a design requirement. In addition, this structure is simple and easy to implement, and can facilitate design and production of the bandpass filter.

With reference to the first aspect, in a feasible implementation, x is greater than or equal to 1 and less than or equal to 2, y is greater than or equal to 0.1 and less than or equal to 1, and n is greater than or equal to 1 and less than or equal to 10.

With reference to the first aspect, in a feasible implementation, the thin-film optical filter is obtained by optimizing a target basic film, the target basic film is determined by the film structure, and the target basic film is Sub| (1.5H0.7L)3 2H (0.7L1.5H)3 L (1.5H0.7L)3 2H (0.7L1.5H)3|Air.

With reference to the first aspect, in a feasible implementation, a material of the high refractive index layer includes tantalum pentoxide $Ta_2O_5$, trititanium pentoxide $Ti_3O_5$, titanium dioxide $TaO_2$, or silicon nitride $Si_3N_4$.

With reference to the first aspect, in a feasible implementation, a material of the low refractive index layer includes silicon dioxide $TiO_2$ or aluminum oxide $Al_2O_3$.

With reference to the first aspect, in a feasible implementation, a material of the substrate includes silicon dioxide $SiO_2$, silicon Si, or transparent high polymer plastic.

With reference to the first aspect, in a feasible implementation, a center wavelength of the bandpass filter is greater than or equal to 880 nm and less than or equal to 920 nm.

With reference to the first aspect, in a feasible implementation, a center wavelength of the bandpass filter is 900 nm, and the optical filter spectrum band ranges 515 nm to 545 nm, 645 nm to 675 nm, and 925 nm to 955 nm, or the optical filter spectrum band ranges 515 nm to 545 nm, 645 nm to 675 nm, 720 nm to 750 nm, and 925 nm to 955 nm.

With reference to the first aspect, in a feasible implementation, the receiving and measuring assembly includes a photoelectric detection component and a processor. The photoelectric detection component is configured to: determine a first electrical signal based on the third detection light, and send the first electrical signal to the processor. The processor is configured to determine the to-be-measured feature value based on the first electrical signal and a second electrical signal, where the second electrical signal is used by the light source emitting assembly to generate the first detection light.

With reference to the first aspect, in a feasible implementation, the optical measurement apparatus is a photoplethysmography PPG sensor, the to-be-measured object is a human body tissue, and the to-be-measured feature value is a human biological parameter.

According to a second aspect, an embodiment of this application provides a bandpass filter. The bandpass filter includes a substrate and a thin-film optical filter disposed on the substrate. A film structure of the thin-film optical filter includes at least two Fabry-Perot cavities disposed in series. Structures of all of the at least two Fabry-Perot cavities are the same. A first Fabry-Perot cavity and a second Fabry-Perot cavity, which are any two adjacent cavities of the at least two Fabry-Perot cavities, are connected through a connection layer. A side that is of the connection layer and that is connected to the first Fabry-Perot cavity and a side that is of the connection layer and that is connected to the second Fabry-Perot cavity each are a low refractive index layer of one basic thickness. A film structure of the first Fabry-Perot cavity is $(xHyL)^n kH(yLxH)^n$, where H represents a high refractive index layer of one basic thickness, L represents a low refractive index layer of one basic thickness, x is an optical thickness coefficient of the high refractive index layer, y is an optical thickness coefficient of the low refractive index layer, xH represents a high refractive index layer of x basic thicknesses, yL represents a low refractive index layer of y basic thicknesses, kH represents a high refractive index material layer of k basic thicknesses, n is a quantity of periodicities of a first membrane stack xHyL and a second membrane stack yLxH, and k is a multiple of 2. The basic thickness is an optical thickness of a quarter of a center wavelength.

The foregoing bandpass filter may have triple bandpass or quadruple bandpass performance, and the optical filter spectrum band of the bandpass filter has a wide coverage area, a large-angle characteristic, a large average transmittance of a passband, a low cut-off rate, and a small average transmittance of each suppression band (that is, a non-optical filter spectrum band). The bandpass filter can effectively filter out light outside the optical filter spectrum band, and has high practicability.

With reference to the second aspect, in a feasible implementation, x is greater than or equal to 1 and less than or equal to 2, y is greater than or equal to 0.1 and less than or equal to 1, and n is greater than or equal to 1 and less than or equal to 10.

With reference to the second aspect, in a feasible implementation, the thin-film optical filter is obtained by optimizing a target basic film, the target basic film is determined by the film structure, and the target basic film is Sub| $(1.5H0.7L)^3$ 2H $(0.7L1.5H)^3$ L $(1.5H0.7L)^3$ 2H $(0.7L1.5H)3$|Air.

With reference to the second aspect, in a feasible implementation, a material of the high refractive index layer includes tantalum pentoxide $Ta_2O_5$, trititanium pentoxide $Ti_3O_5$, titanium dioxide $TaO_2$, or silicon nitride $Si_3N_4$.

With reference to the second aspect, in a feasible implementation, a material of the low refractive index layer includes silicon dioxide $TiO_2$ or aluminum oxide $Al_2O_3$.

With reference to the second aspect, in a feasible implementation, a material of the substrate includes silicon dioxide $SiO_2$, silicon Si, or transparent high polymer plastic.

With reference to the second aspect, in a feasible implementation, a center wavelength of the bandpass filter is greater than or equal to 880 nm and less than or equal to 920 nm.

With reference to the second aspect, in a feasible implementation, a center wavelength of the bandpass filter is 900 nm, and the optical filter spectrum band ranges 515 nm to 545 nm, 645 nm to 675 nm, and 925 nm to 955 nm, or the optical filter spectrum band ranges 515 nm to 545 nm, 645 nm to 675 nm, 720 nm to 750 nm, and 925 nm to 955 nm.

According to a third aspect, an embodiment of this application provides an optical measurement method. The method is applicable to the optical measurement apparatus according to the first aspect. The optical measurement apparatus includes a light source emitting assembly, a bandpass filter, and a receiving and measuring assembly. The bandpass filter is disposed between a to-be-measured object and the receiving and measuring assembly. An optical filter spectrum band of the bandpass filter includes a light source operating spectrum band of the light source emitting assembly. The method includes: emitting first detection light to the to-be-measured object through the light source emitting assembly; emitting third detection light to the receiving and measuring assembly through the bandpass filter based on second detection light, where the second detection light includes light reflected or transmitted by the to-be-measured object based on the first detection light; and determining a to-be-measured feature value of the to-be-measured object based on the first detection light and the third detection light through the receiving and measuring assembly.

In the foregoing implementation, the bandpass filter disposed between the receiving and measuring assembly and the to-be-measured object may filter out interference light emitted to the receiving and measuring assembly, so that the receiving and measuring assembly receives only a valid optical signal, and the interference signal is eliminated radically. Therefore, the foregoing optical measurement method has high measurement accuracy and high practicability.

With reference to the third aspect, in a feasible implementation, the receiving and measuring assembly includes a photoelectric detector and a processor. A first electrical signal may be determined based on the third detection light through the photoelectric detector, and the first electrical signal is sent to the processor. The to-be-measured feature value may be determined based on the first electrical signal and a second electrical signal through the processor. The second electrical signal is used by the light source emitting assembly to generate the first detection light.

According to a fourth aspect, an embodiment of this application provides an electronic device. The electronic device includes the optical measurement apparatus according to any implementation of the first aspect, a power supply, and a controller, where the optical measurement apparatus, the power supply, and the controller are coupled to each other. In actual operation, the power supply is configured to supply power to the optical measurement apparatus and the controller. The controller is configured to control the optical measurement apparatus to perform corresponding detection on a to-be-measured object, to obtain a corresponding to-be-measured feature value.

With reference to the fourth aspect, in a first possible implementation, the electronic device further includes a fastening assembly. The fastening assembly is mainly used for fastening between the electronic device and the to-be-measured object.

With reference to the fourth aspect, in a feasible implementation, the electronic device is specifically a wearable device (like a smartwatch), and is mainly configured to detect a human biological parameter. Herein, the human biological parameter may include a pulse wave, a heart rate, blood oxygen saturation, blood pressure, and the like.

With reference to the fourth aspect, in a feasible implementation, the controller and the processor in the optical measurement assembly may be a same component, or the processor may be included inside the controller.

The solutions provided in the second aspect to the fourth aspect are used to implement or cooperate to implement the optical measurement apparatus provided in any implementation of the first aspect. Therefore, the solutions can achieve same or corresponding beneficial effect as the first aspect. Details are not described herein again.

In conclusion, according to the optical measurement apparatus and method provided in embodiments of this application, interference light can be effectively reduced or even eliminated, and measurement accuracy and practicability of the optical measurement apparatus can be improved.

**BRIEF DESCRIPTION OF DRAWINGS**

FIG. 1 is a diagram of a structure of an optical measurement apparatus according to an embodiment of this application;

FIG. 2 is a diagram of a structure of a bandpass filter according to an embodiment of this application;

FIG. 3 is a diagram of a film structure of a thin-film optical filter according to an embodiment of this application;

FIG. 4 is a diagram of a transmission passband curve of a single bandpass optical filter film according to an embodiment of this application;

FIG. 5 is a diagram of a transmittance curve of a thin-film optical filter according to an embodiment of this application;

FIG. 6 is a diagram of another transmittance curve of a thin-film optical filter according to an embodiment of this application;

FIG. 7 is a diagram of another structure of an optical measurement apparatus according to an embodiment of this application;

FIG. 8 is a diagram of still another structure of an optical measurement apparatus according to an embodiment of this application;

FIG. 9 is a schematic flowchart of an optical measurement method according to an embodiment of this application; and

FIG. 10 is a diagram of a structure of an electronic device according to an embodiment of this application.

**DESCRIPTION OF EMBODIMENTS**

The following clearly and completely describes technical solutions in embodiments of this application with reference to accompanying drawings in embodiments of this application.

An existing optical measurement apparatus usually performs, according to a built-in filter algorithm of the optical measurement apparatus, digital signal processing on a measurement signal obtained by the optical measurement apparatus, to filter out an interference signal in the measurement signal, so as to improve measurement accuracy of the optical measurement apparatus. However, this imposes a higher requirement on a data processing capability of the optical measurement apparatus, and it is difficult to completely filter out the interference signal. As a result, the measurement accuracy of the existing optical measurement apparatus is not ideal, and practicability is poor.

Therefore, a technical problem to be resolved in this application is how to simply and effectively remove an interference signal, so as to improve measurement accuracy and practicability of an optical measurement apparatus.

**Embodiment 1**

To resolve the foregoing technical problems, this application provides an optical measurement apparatus. The optical measurement apparatus includes a light source emitting assembly, a bandpass filter, and a receiving and measuring assembly. The bandpass filter is disposed between a to-be-measured object and the receiving and measuring assembly. An optical filter spectrum band of the bandpass filter includes a light source operating spectrum band corresponding to the light source emitting assembly. In actual operation, signal light that is sent by the light source emitting assembly and reflected or transmitted by the to-be-measured object may pass through the bandpass filter at a high transmittance, and arrive at the receiving and measuring assembly; and interference light (that is, interference signals) of a band outside the light source operating spectrum band cannot be received by the receiving and measuring assembly through the bandpass filter. In this way, the receiving and measuring assembly can receive only the signal light that is sent by the light source emitting assembly and that is emitted or transmitted by the to-be-measured object, and does not receive the interference light. This radically prevents an interference signal from entering the optical measurement apparatus. This manner is simple and effective, and can significantly improve measurement accuracy and practicability of the optical measurement apparatus.

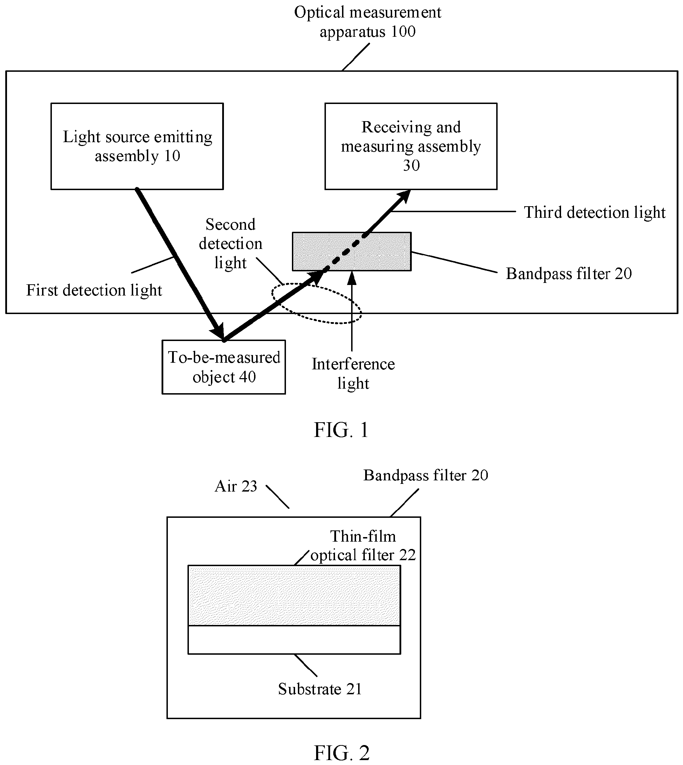

The following describes in detail a structure and an operating principle of the optical measurement apparatus provided in this application with reference to FIG. 1 to FIG. 7.

Refer to FIG. 1. FIG. 1 is a diagram of a structure of an optical measurement apparatus according to an embodiment of this application. As shown in FIG. 1, the optical measurement apparatus 100 may include a light source emitting assembly 10, a bandpass filter 20, and a receiving and measuring assembly 30. The bandpass filter 20 is disposed between a to-be-measured object 40 and the receiving and measuring assembly 30. In other words, the bandpass filter 20 is disposed on a spatial optical path between the to-be-measured object 40 and the receiving and measuring assembly 30, so that light reflected or transmitted by the to-be-measured object 40 first passes through the bandpass filter 20 and then is received by the receiving and measuring assembly 30. An optical filter spectrum band of the bandpass filter 20 includes a light source operating spectrum band of the light source emitting assembly 10. Herein, the optical filter spectrum band is a spectrum band of a light wave that can pass through the bandpass filter 20. For example, if the optical filter spectrum band of the bandpass filter 20 is from 515 nm to 545 nm, the bandpass filter 20 has a very high transmittance for a light wave whose wavelength is from 515 nm to 545 nm, and has a very low transmittance for a light wave whose wavelength is outside a range of 515 nm to 545 nm. The light source operating spectrum band is a wavelength range of a light wave that can be emitted by the light source emitting assembly 10. For example, if the light source operating spectrum band of the light source emitting assembly 10 is from 517 nm to 543 nm, it indicates that the light source emitting assembly 10 may emit a light beam with a wavelength of 517 nm to 543 nm. It should be understood that the bandpass filter 20 provided in this application generally has a plurality of optical filter spectrum bands. Preferably, the bandpass filter 20 has three or four optical filter spectrum bands. Similarly, the light source operating spectrum bands provided in this application should one-to-one correspond to the optical filter spectrum bands. Preferably, there are also three or four light source operating spectrum bands. It should be further noted herein that, the foregoing described optical filter spectrum band includes the light source operating spectrum band, and this may be understood as that a range of the optical filter spectrum band is slightly greater than that of the light source operating spectrum band. For example, it may be designed that a difference between an upper limit value of the optical filter spectrum band and an upper limit value of the light source operating spectrum band and a difference between a lower limit value of the optical filter spectrum band and a lower limit value of the light source operating spectrum band are equal to a preset difference. For example, if the optical filter spectrum band of the bandpass filter 20 is from 515 nm to 545 nm, and a preset difference is 3 nm, the light source operating spectrum band may be from 512 nm to 542 nm. The foregoing is merely an example. In an actual implementation, as long as it can be ensured that the optical filter spectrum band includes the light source operating spectrum band, a relationship between the optical filter spectrum band and the light source operating spectrum band is not specifically limited in this application. Certainly, when there are a plurality of optical filter spectrum bands, it also needs to be ensured that each optical filter spectrum band includes a corresponding light source operating spectrum band.

In actual operation, the optical measurement apparatus 100 is mainly configured to measure one or more to-be-measured feature values of the to-be-measured object 40. Herein, the to-be-measured feature value of the to-be-measured object 40 may be specifically an attribute parameter value like a size or a color of the to-be-measured object 40. Specifically, the light source emitting assembly 10 may be configured to generate and emit a light beam (for ease of differentiation, first detection light is used as a substitute for description below) to the to-be-measured object 40. The to-be-measured object 40 may reflect or refract the first detection light, to emit another light beam (for ease of differentiation, fourth detection light is used as a substitute for description below) to the bandpass filter 20. Herein, the fourth detection light is light reflected or refracted by the to-be-measured object 40 based on the first detection light. In addition, the bandpass filter 20 may be configured to emit another light beam (for ease of differentiation, third detection light is used as a substitute for description below) to the receiving and measuring assembly 30 based on light (for ease of differentiation, second detection light is used as a substitute for description below) received by the bandpass filter 20. It may be understood that the third detection light is obtained after the bandpass filter 20 performs optical filter on the second detection light. The second detection light is light received by the bandpass filter 20 when the first detection light irradiates the to-be-measured object 40. The second detection light includes the fourth detection light, and may include interference light whose wavelength is outside the light source operating spectrum band. Herein, the interference light may include ambient light existing between the to-be-measured object 40 and the receiving and measuring assembly 30, and light reflected or transmitted by the to-be-measured object 40 based on the ambient light. A main function of the bandpass filter 20 is to filter out the interference light, and emit the fourth detection light to the receiving and measuring assembly 30 as completely as possible. The receiving and measuring assembly 30 is configured to receive the third detection light, and further determine the to-be-measured feature value of the to-be-measured object based on the third detection light and the first detection light.

It should be noted herein that in an actual implementation, the fourth detection light may be light reflected by the to-be-measured object 40 based on the first detection light (in other words, the optical measurement apparatus 100 is a reflective component), or may be light transmitted by the to-be-measured object 40 based on the first detection light (in other words, the optical measurement apparatus 100 is a transmissive component). A difference between the two cases is whether the receiving and measuring assembly 30 is in a direction of the reflected light or a direction of the transmitted light from the to-be-measured object 40. This does not affect connection relationships and functions of components in the optical measurement apparatus 100. Therefore, in this embodiment of this application, a structure and a function of the optical measurement apparatus 100 are described by using an example in which the optical measurement apparatus 100 is a reflective component. Details of a case in which the optical measurement apparatus 100 is a transmissive component are not described again.

In the foregoing implementation, one bandpass filter 20 whose optical filter spectrum band includes the light source operating spectrum band of the light source emitting assembly 10 is disposed between the receiving and measuring assembly 30 of the optical measurement apparatus 100 and the to-be-measured object 40. In this way, interference light emitted to the receiving and measuring assembly 30 can be filtered out through the bandpass filter 20, so that the receiving and measuring assembly 30 may receive only a valid optical signal that is sent by the light source emitting assembly 10 and that is reflected or transmitted by the to-be-measured object 40, and the interference signal is eliminated radically. This manner is simple and effective, and can significantly improve measurement accuracy and practicability of the optical measurement apparatus 100.

In some feasible implementations, refer to FIG. 2. FIG. 2 is a diagram of a structure of a bandpass filter according to an embodiment of this application. As shown in FIG. 2, the bandpass filter 20 may include a substrate 21 and a thin-film optical filter 22 disposed on the substrate 21. In other words, one side of the thin-film optical filter 22 is in contact with the substrate 21, and the other side of the thin-film optical filter 22 is in contact with air 23.

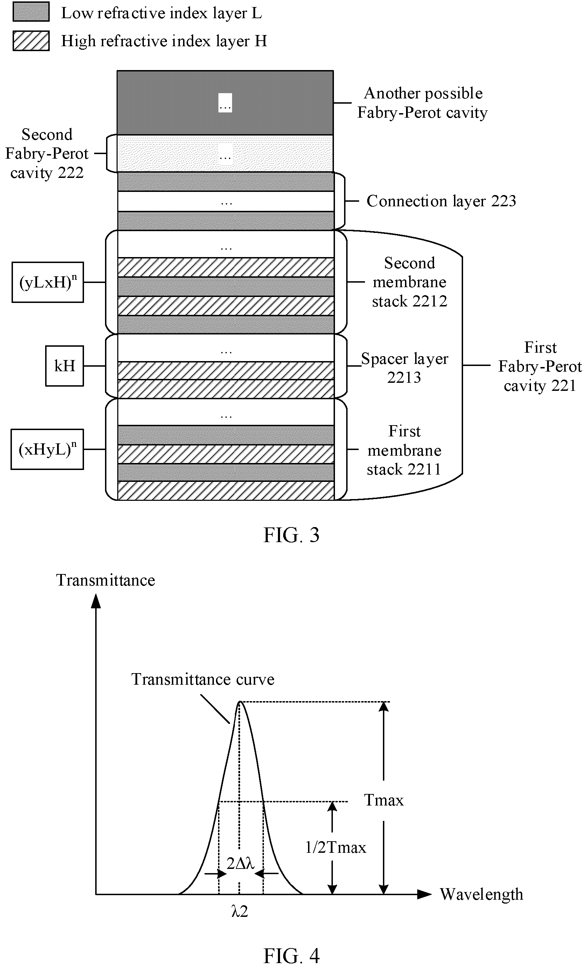

Further, refer to FIG. 3. FIG. 3 is a diagram of a film structure of a thin-film optical filter according to an embodiment of this application. As shown in FIG. 3, the film structure of the thin-film optical filter 22 may include at least two Fabry-Perot cavities (a first Fabry-Perot cavity 221, a second Fabry-Perot cavity 222, and another possible Fabry-Perot cavity shown in FIG. 3) that are disposed in series. Structures of all of the at least two Fabry-Perot cavities are the same. Any two adjacent Fabry-Perot cavities (for example, the first Fabry-Perot cavity 221 and the second Fabry-Perot cavity 222 in FIG. 3) in the at least two Fabry-Perot cavities are connected through a connection layer (for example, a connection layer 223 in FIG. 3). A side that is of the connection layer 223 and that is connected to the first Fabry-Perot cavity 221 and a side that is of the connection layer 223 and that is connected to the second Fabry-Perot cavity 222 each are a low refractive index layer of one basic thickness. It should be noted herein that, in an optional implementation, as shown in FIG. 3, the connection layer 223 may include at least two low refractive index layers (where the two low refractive index layers each are of one basic thickness). In this case, one low refractive index layer in the connection layer 223 is connected to the first Fabry-Perot cavity 221, and the other low refractive index layer is connected to the second Fabry-Perot cavity 222. One or more overlapping low refractive index layers and/or high refractive index layers may exist between the two low refractive index layers, or the two low refractive index layers may be directly connected. However, in another optional implementation, the connection layer 223 may include only a low refractive index layer of one basic thickness. In this case, one side of the low refractive index layer is connected to the first Fabry-Perot cavity 221, and the other side is connected to the second Fabry-Perot cavity 222. It should be understood that a structure of the connection layer 223 may be determined according to a specific design requirement, provided that the foregoing connection manner between the connection layer and any two adjacent Fabry-Perot cavities can be met.

Because the structures of all Fabry-Perot cavities are the same, the following uses the first Fabry-Perot cavity 221 as an example to describe the structure of the plurality of Fabry-Perot cavities that are disposed in series. As shown in FIG. 3, a film structure of the first Fabry-Perot cavity 221 may be represented as $(xHyL)^n kH(yLxH)^n$ (which may also be referred to as a film formula). H represents a high refractive index layer of one basic thickness (that is, a thin film layer made of a high refractive index material). It should be noted herein that the basic thickness is an optical thickness of a quarter of a center wavelength (quarter-wave, where it is assumed that the center wavelength is $\lambda_1$ herein), and the center wavelength $\lambda_1$ is a center wavelength designed for the thin-film optical filter 22. It may alternatively be understood that one basic thickness is equal to $\lambda_1/4$. L represents a low refractive index layer of one basic thickness (that is, a thin film layer made of a high/low refractive index material). x is an optical thickness coefficient of the high refractive index layer, and y is an optical thickness coefficient of the low refractive index layer. Herein, values of x and y are adjustable. xH represents a high refractive index layer of x basic thicknesses, and yL represents a low refractive index layer of y basic thicknesses. kH represents a low refractive index material layer of k basic thicknesses. Herein, k is a multiple of 2. n is a quantity of periodicities (which may also be referred to as a group number) of a first membrane stack 2211 (namely, xHyL) and a second membrane stack 2212 (namely, yLxH).

The film structure of the first Fabry-Perot cavity is $(xHyL)^n kH(yLxH)^n$. H represents the high refractive index layer of one basic thickness, L represents the low refractive index layer of one basic thickness, x is the optical thickness coefficient of the high refractive index layer, y is the optical thickness coefficient of the low refractive index layer, xH represents the high refractive index layer of x basic thicknesses, yL represents the low refractive index layer of y basic thicknesses, kH represents the high refractive index material layer of k basic thicknesses, n is the quantity of periodicities of the first membrane stack xHyL and the second membrane stack yLxH, and k is a multiple of 2. The basic thickness is the optical thickness of a quarter of the center wavelength. In other words, the first Fabry-Perot cavity 221 may include the first membrane stack 2211, the second membrane stack 2212, and a spacer layer 2213. An initial structure of the first membrane stack 2211 is $(xHyL)^n$. In other words, sequences of n groups of xHyL are repeated and alternately stacked. The spacer layer 2213 may also be referred to as a space layer, and a structure of the spacer layer 2213 is a high refractive index layer of k basic thicknesses. An initial structure of the second membrane stack 2212 is $(yLxH)^n$. In other words, sequences of n groups of yLxH are repeated and alternately stacked.

It should be understood that, with reference to the foregoing descriptions of the film structure of the thin-film optical filter 22 and the structure of each Fabry-Perot cavity included in the thin-film optical filter 22, when the film structure of the thin-film optical filter 22 includes only two Fabry-Perot cavities that are disposed in series and the connection layer is a low refractive index layer of one basic thickness, the film structure of the thin-film optical filter 22 may be represented as $Sub|(xHyL)^n kH(yLxH)^n L(xHyL)^n kH(yLxH)^n|Air$. Herein, Sub is the substrate 21 described above, and Air is the air 23.

In the foregoing implementation, the bandpass filter 20 is implemented by disposing Fabry-Perot cavities in series. In this way, a bandpass gradient of the bandpass filter 20 can be increased, and a rectangularity of a passband curve is more obvious, so as to ensure that a width and a transmittance of the optical filter spectrum band of the bandpass filter 20 and a cut-off rate of the bandpass filter 20 can meet a design requirement. In addition, this structure is simple and easy to implement, and can facilitate design and production of the bandpass filter 20.

The following describes in detail a structure design principle of the bandpass filter 20 with reference to an optical characteristic of the Fabry-Perot cavity.

Generally, one of the most basic design formulas of a single bandpass optical filter film (herein, it is assumed that the single bandpass optical filter film is S1, and there is only one optical filter spectrum band of the single bandpass optical filter film) is a single-cavity design. In other words, only one Fabry-Perot cavity (herein, it is assumed that the Fabry-Perot cavity is F-P1) is used to implement the single bandpass optical filter film S1. It is assumed that a film structure of the single bandpass optical filter film S1 is Sub|(HL)$^n$ 2H (LH)$^n$|Air. Herein, for descriptions of parameters in the film formula, refer to the foregoing descriptions. Details are not described herein again. Refer to FIG. 4. FIG. 4 is a diagram of a transmission passband curve of a single bandpass optical filter film according to an embodiment of this application. As shown in FIG. 4, optical filter characteristic parameters of the single bandpass optical filter film S1 mainly include a center wavelength $\lambda_2$, a peak transmittance ($T_{max}$), a passband half width $2\Delta\lambda$, a relative passband half width $2\Delta\lambda/\lambda_2$, and the like.

It can be learned from an effective interface method that, a thin film transmittance (herein, it is assumed that the thin film transmittance is T) of the single bandpass optical filter film S1 should meet the following formula (1):

$$T = T_0/(1 + F1\,sin^2\,\theta)\ \ (1)$$

Herein, $T_0$ is a center wavelength transmittance of the single bandpass optical filter film S1, F1 is a theoretical function of the Fabry-Perot cavity F-P1, and $\theta$ is an incident angle of incident light of the single bandpass optical filter film S1.

Further, the center wavelength transmittance $T_0$ should meet the following formula (2):

$$T_0 = \frac{T_1 T_2}{\left(1 - \sqrt{R_1 R_2}\right)^2}\ \ (2)$$

The theoretical function F1 of the Fabry-Perot cavity F-P1 should meet the following formula (3):

$$F1 = \frac{4\sqrt{R_1 R_2}}{\left(1 - \sqrt{R_1 R_2}\right)^2}\ \ (3)$$

The incident angle $\theta$ should meet the following formula (4):

$$\theta = \frac{1}{2}\left(\varphi_1 + \varphi_2 - 2\delta\right)\ \ (4)$$

Herein, $T_1$ is a transmittance of a reflection layer (where the reflection layer is a membrane stack between a membrane stack (HL)$^n$ and a membrane stack (LH)$^n$ according to a film formula, and it is assumed that the reflection layer is the membrane stack (HL)$^n$ herein) in the Fabry-Perot cavity F-P1, and $R_1$ is a reflective index of the reflection layer. $T_2$ is a transmittance of another reflection layer (where the reflection layer is the membrane stack (LH)$^n$ with reference to the foregoing assumption) in the Fabry-Perot cavity F-P1, and $R_2$ is a reflective index of the reflection layer. $\varphi_1$ is a reflection phase of the membrane stack (HL)$^n$, and $\varphi_2$ is a reflection phase of the membrane stack (LH)$^n$. $\delta$ is a phase thickness of a spacer layer (which may also be referred to as a connection layer) of the Fabry-Perot cavity F-P1, and the phase thickness meets a formula $\delta = \frac{2\pi}{\lambda_2} \times n1 \times d$ . $n1$ is a refractive index of the spacer layer of the Fabry-Perot cavity F-P1, and d is a physical thickness of the spacer layer of the Fabry-Perot cavity F-P1.

With reference to the foregoing formula (1) to formula (4), it can be learned that the passband half width $2\Delta\lambda$ of the single bandpass optical filter film S1 may meet the following formula (5):

$$2\Delta\lambda = \frac{2\lambda_2}{m_1 \pi} arcsin\left(\frac{1}{\sqrt{F}}\right) = \frac{2\lambda_2}{m_1 \pi} arcsin\left(\frac{1 - \bar{R}}{2\sqrt{R}}\right)\ \ (5)$$

The relative half width $2\Delta\lambda/\lambda_2$ of the single bandpass optical filter film S1 may meet the following formula (6):

$$2\Delta\lambda/\lambda_2 = \frac{2}{m_1 \pi} arcsin\left(\frac{1 - \bar{R}}{2\sqrt{R}}\right)\ \ (6)$$

$\overline{R}$ is an average reflective index of the two reflection layers of the Fabry-Perot cavity F-P1, and the average reflective index meets a formula $\overline{R} = \sqrt{R_1 R_2}$. $m_1$ is an interference level of the Fabry-Perot cavity F-P1.

With reference to the foregoing formula (5) or formula (6), it can be learned that the reflective indexes and the interference levels of the two reflection layers in the Fabry-Perot cavity F-P1 determine the passband half width $2\Delta\lambda$ of the single bandpass optical filter film S1. Higher reflective indexes of the two reflection layers indicate a thicker spacer layer (that is, a larger interference level m) and a larger bandpass gradient of the single bandpass optical filter film S1. However, when the interference level m is increased, a passband bandwidth of the single bandpass optical filter film S1 may be compressed, and a secondary peak may be generated on a long-wave side of a main peak of the transmittance curve of the single bandpass optical filter film S1. Therefore, when the thin-film optical filter 22 is obtained through optimization based on the single bandpass optical filter film S1, the passband half width and the cut-off rate of the thin-film optical filter 22 need to be ensured on the premise that a plurality of bandpass transmittances are improved as much as possible.

It can be learned from existing research that, replacing a metal reflection layer in the Fabry-Perot cavity with a multilayer dielectric reflection film can reduce light absorption of the Fabry-Perot cavity, so that performance of an optical filter film is improved. If a bandpass optical filter film S2 is implemented based on a Fabry-Perot cavity F-P2 (also referred to as an all-dielectric Fabry-Perot cavity) of a multi-layer dielectric emission film, a passband half width (for ease of differentiation, it is assumed that passband half width is $2\Delta\lambda_3$ herein) of the bandpass optical filter film S2 is calculated as follows. Herein, if two reflection layers of the Fabry-Perot cavity F-P2 have no absorption and scattering loss (that is, the reflective index is high enough), and the two reflection films are completely symmetric, reflective indexes, transmittances, and optical admittances of the two reflection layers are the same, and it is assumed that the reflective indexes, the transmittances, and the optical admittances of the two reflection layers are $R_{12}$, $T_{12}$, and $Y_{12}$ respectively.

A theoretical function F2 of the Fabry-Perot cavity F-P2 should meet the following formula (7):

$$F = \frac{4R_{12}}{(1-R_{12})^2} \approx \frac{4}{T_{12}^2} \ (7)$$

A passband half width $2\Delta\lambda_3$ of the bandpass optical filter film S2 may meet the following formula (8):

$$2\Delta\lambda_3 = \frac{2\lambda_4}{m_2\pi} arcsin\left(\frac{T_{12}}{2}\right) \ (8)$$

Herein, $\lambda_4$ is a center wavelength of the bandpass optical filter film S2, and $m_2$ is an interference level of the Fabry-Perot cavity F-P2.

If a connection layer of the Fabry-Perot cavity F-P2 is not counted, and if a quantity of high refractive index layers in each reflection layer of the Fabry-Perot cavity F-P2 is h, for the connection layer of the Fabry-Perot cavity F-P2 that uses a high refractive index material, an optical admittance $Y_{12}$ meets the following formula (9):

$$Y_{12} = \frac{n_L^{2h}}{n_H^{2h}} n_g \ (9)$$

Herein, $n_L$ is a refractive index of a low refractive index layer of the Fabry-Perot cavity F-P2, $n_H$ is a refractive index of a high refractive index layer of the Fabry-Perot cavity F-P2, and $n_g$ is a refractive index of a substrate of the Fabry-Perot cavity F-P2.

For the connection layer of the Fabry-Perot cavity F-P2, a reflective index $R_{12}$ meets the following formula (10):

$$R_{12} = (n_H - Y_{12})^2/(n_H + Y_{12})^2 = (1 - Y_{12}/n_H)^2/(1 + Y_{12}/n_H)^2 \ (10)$$

When each reflection layer in the Fabry-Perot cavity F-P2 includes a sufficient quantity of high refractive index layers, the transmittance $T_{12}$ may meet the following formula (11):

$$T_{12} \approx 4Y_{12}/n_H = 4n_L^{2h}n_g/n_H^{2h+1} \ (11)$$

It can be learned by adding the formula (11) to the foregoing formula (8) that, when the connection layer of the Fabry-Perot cavity F-P2 uses a high refractive index material, the passband half width $2\Delta\lambda_3$ of the Fabry-Perot cavity F-P2 meets the following formula (12):

$$2\Delta\lambda_3 = \frac{2\lambda_4}{m_2\pi}arcsin(2n_L^{2\mathrm{h}}n_g/n_H^{2\mathrm{h}+1}) \approx 4\lambda_4 n_L^{2\mathrm{h}}n_g/(m_2\pi\, n_H^{2\mathrm{h}+1}) \quad (12)$$

When the connection layer of the Fabry-Perot cavity F-P2 uses a low refractive index material, the passband half width $2\Delta\lambda_3$ of the Fabry-Perot cavity F-P2 meets the following formula (13):

$$2\Delta\lambda_3 = \frac{2\lambda_4}{m_2\pi}arcsin(2n_L^{2\mathrm{h}-1}n_g/n_H^{2\mathrm{h}}) \approx 4\lambda_4 n_L^{2\mathrm{h}-1}n_g/(m_2\pi\, n_H^{2\mathrm{h}}) \quad (13)$$

With reference to the foregoing inference, it can be learned that, to approximately implement multi-passband transmission, during initial design, the bandpass filter may be designed to have a structure of a single Fabry-Perot cavity, and a film structure of the bandpass filter may be Sub|(xHyL)$^n$2H (yLxH)$^n$)|Air. A location of a transmission band may be adjusted by adjusting a ratio of x to y and the quantity of periodicities n, so that the bandpass filter has a basic shape of a plurality of transmission peaks.

Further, because the multi-bandpass filter includes only one Fabry-Perot cavity, a quantity of film layers of the multi-bandpass filter is small, and a structure of the multi-bandpass filter is simple, the multi-bandpass filter cannot be truly implemented. It can be learned with reference to existing research that, theoretically, increasing a quantity of Fabry-Perot cavities that are disposed in series makes a rectangularity of a passband curve of the bandpass filter more obvious. Therefore, based on the structure of one Fabry-Perot cavity, a plurality of Fabry-Perot cavities (that is, a multi-half-wave model is introduced) may be disposed in series to improve the rectangularity of the passband curve, increase the bandpass gradient, and deepen a cutoff degree, so that a bandpass filter that can truly implement multi-bandpass can be obtained through optimization. Therefore, the thin-film optical filter 22 provided in this application is formed by a plurality of Fabry-Perot cavities in series, and can implement multi-bandpass performance through optimization.

In some feasible implementations, x is greater than or equal to 1 and less than or equal to 2, y is greater than or equal to 0.1 and less than or equal to 1, and n is greater than or equal to 1 and less than or equal to 10.

In some feasible implementations, a center wavelength of the bandpass filter 20 may be greater than or equal to 880 nm and less than or equal to 920 nm.

In some feasible implementations, a material of the high refractive index layer H in the bandpass filter 20 may include tantalum pentoxide $Ta_2O_5$, trititanium pentoxide $Ti_3O_5$, titanium dioxide $TaO_2$, silicon nitride $Si_3N_4$, or the like.

In some feasible implementations, a material of the low refractive index layer in the bandpass filter 20 may include silicon dioxide $TiO_2$ or aluminum oxide $Al_2O_3$.

In some feasible implementations, a material of the substrate 21 may include silicon dioxide $SiO_2$, silicon Si, or transparent high polymer plastic (like polyethylene terephthalate PET and polyimide PA).

It should be understood herein that the foregoing descriptions of the materials of the high refractive index layer H, the low refractive index layer L, and the substrate 21 in the bandpass filter 20 are examples and are not exhaustive. In an actual implementation, the high refractive index layer H, the low refractive index layer L, and the substrate 21 in the bandpass filter 20 may alternatively use other types of materials, provided that the structure, that is, a performance requirement, of the bandpass filter 20 can be met. This is not specifically limited in this application.

It should be noted that, in an actual implementation, if a film structure of the thin-film optical filter 22 is designed to be Sub|(xHyL)$^n$2H(yLxH)$^n$L(xHyL)$^n$2H(yLxH)$^n$|Air, specific values of x, y, and n may be subsequently set based on the performance requirement of the bandpass filter 20, so as to obtain a basic film that can be applied to software optimization. After the basic film is obtained, optimization may be performed by using professional optical thin film design software (like essential macleod and optilayer), to obtain specific structure parameters of the thin-film optical filter 22. These specific structure parameters may include a quantity of low refractive index layers, a quantity of high refractive index layers, and physical thicknesses of low refractive index layers and high refractive index layers of the thin-film optical filter 22. Finally, the specific structure parameters of the thin-film optical filter 22 are obtained based on software, so that the thin-film optical filter 22 can be generated on the substrate 21, to obtain the bandpass filter 20.

For example, a center wavelength of the bandpass filter 20 (that is, a center wavelength $\lambda_1$ of the thin-film optical filter 22) may be designed as 900 nm. If a value of x is 1.5, a value of y is 0.7, and a value of n is 3, a basic film (for ease of differentiation, a target basic film is used as a substitute for description below) Sub| (1.5H0.7L)$^3$ 2H (0.7L1.5H)$^3$ L (1.5H0.7L)$^3$ 2H (0.7L1.5H)$^3$|Air may be obtained. Subsequently, the bandpass filter 20 may be obtained through optimization based on the target basic film Sub| (1.5H0.7L)$^3$ 2H (0.7L1.5H)$^3$ L (1.5H0.7L)$^3$ 2H (0.7L1.5H)3|Air.

In some feasible implementations, the bandpass filter 20 may be specifically a triple bandpass filter, and an optical filter spectrum band of the bandpass filter 20 may specifically range 515 nm to 545 nm (which is assumed as a first optical filter spectrum band), 645 nm to 675 nm (which is assumed as a second optical filter spectrum band), and 925 nm to 955 nm (which is assumed as a third optical filter spectrum band). It should be understood that, in this case, there may also be three light source operating spectrum bands of the light source operating assembly 10, and the three light source operating spectrum bands may specifically include a first light source operating spectrum band, a second light source operating spectrum band, and a third light source operating spectrum band. The first optical filter spectrum band may include the first light source operating spectrum band, the second optical filter spectrum band may include the second light source operating spectrum band, and the third optical filter spectrum band may include the third light source operating spectrum band.

For example, when optimization is performed based on the target basic film Sub| $(1.5H0.7L)^3$ 2H $(0.7L1.5H)^3$ L $(1.5H0.7L)^3$ 2H $(0.7L1.5H)^3$|Air, the material of the substrate 21 may be set as silicon dioxide $SiO_2$, the material of the high refractive index layer H may be set as tantalum pentoxide $Ta_2O_5$, and the material of the low refractive index layer H may be set as $SiO_2$. After software optimization, the quantity of low refractive index layers, the quantity of high refractive index layers, and the physical thicknesses of low refractive index layers and high refractive index layers of the thin-film optical filter 22 may be determined. For example, there are a total of 84 layers of the low refractive index layers and the high refractive index layers of the thin-film optical filter 22. For details, refer to Table 1.

Table 1 Film layer structure of a thin-film optical filter 22

| Layer number | Material | Optical thickness | Physical thickness (nm) |
| --- | --- | --- | --- |
| 1 | $Ta_2O_5$ | 0.313622 | 133.83 |
| 2 | $SiO_2$ | 0.375719 | 232.92 |
| 3 | $Ta_2O_5$ | 0.279244 | 119.16 |
| 4 | $SiO_2$ | 0.025858 | 16.03 |
| 5 | $Ta_2O_5$ | 0.044057 | 18.8 |
| 6 | $SiO_2$ | 0.284483 | 176.36 |
| 7 | $Ta_2O_5$ | 0.26366 | 112.51 |
| 8 | $SiO_2$ | 0.455599 | 282.44 |
| 9 | $Ta_2O_5$ | 0.243366 | 103.85 |
| 10 | $SiO_2$ | 0.310873 | 192.72 |
| 11 | $Ta_2O_5$ | 0.25745 | 109.86 |
| 12 | $SiO_2$ | 0.218234 | 135.29 |
| 13 | $Ta_2O_5$ | 0.257379 | 109.83 |
| 14 | $SiO_2$ | 0.058716 | 36.4 |
| 15 | $Ta_2O_5$ | 0.12146 | 51.83 |
| 16 | $SiO_2$ | 0.252835 | 156.74 |
| 17 | $Ta_2O_5$ | 0.336189 | 143.46 |
| 18 | $SiO_2$ | 0.313245 | 194.19 |
| 19 | $Ta_2O_5$ | 0.190357 | 81.23 |
| 20 | $SiO_2$ | 0.122562 | 75.98 |
| 21 | $Ta_2O_5$ | 0.084832 | 36.2 |
| 22 | $SiO_2$ | 0.189053 | 117.2 |
| 23 | $Ta_2O_5$ | 0.304154 | 129.79 |
| 24 | $SiO_2$ | 0.405448 | 251.35 |
| 25 | $Ta_2O_5$ | 0.173789 | 74.16 |
| 26 | $SiO_2$ | 0.177778 | 110.21 |
| 27 | $Ta_2O_5$ | 0.371669 | 158.6 |
| 28 | $SiO_2$ | 0.2022 | 125.35 |
| 29 | $Ta_2O_5$ | 0.031261 | 13.34 |
| 30 | $SiO_2$ | 0.081428 | 50.48 |
| 31 | $Ta_2O_5$ | 0.149863 | 63.95 |

(continued)

| Layer number | Material | Optical thickness | Physical thickness (nm) |
|---|---|---|---|
| 32 | $SiO_2$ | 0.124562 | 77.22 |
| 33 | $Ta_2O_5$ | 0.157549 | 67.23 |
| 34 | $SiO_2$ | 0.181633 | 112.6 |
| 35 | $Ta_2O_5$ | 0.279548 | 119.29 |
| 36 | $SiO_2$ | 0.265175 | 164.39 |
| 37 | $Ta_2O_5$ | 0.055188 | 23.55 |
| 38 | $SiO_2$ | 0.028713 | 17.8 |
| 39 | $Ta_2O_5$ | 0.218924 | 93.42 |
| 40 | $SiO_2$ | 0.182988 | 113.44 |
| 41 | $Ta_2O_5$ | 0.081973 | 34.98 |
| 42 | $SiO_2$ | 0.157195 | 97.45 |
| 43 | $Ta_2O_5$ | 0.251872 | 107.48 |
| 44 | $SiO_2$ | 0.222089 | 137.68 |
| 45 | $Ta_2O_5$ | 0.199215 | 85.01 |
| 46 | $SiO_2$ | 0.331972 | 205.8 |
| 47 | $Ta_2O_5$ | 0.185155 | 79.01 |
| 48 | $SiO_2$ | 0.195473 | 121.18 |
| 49 | $Ta_2O_5$ | 0.072318 | 30.86 |
| 50 | $SiO_2$ | 0.066185 | 41.03 |
| 51 | $Ta_2O_5$ | 0.069178 | 29.52 |
| 52 | $SiO_2$ | 0.167067 | 103.57 |
| 53 | $Ta_2O_5$ | 0.205425 | 87.66 |
| 54 | $SiO_2$ | 0.279596 | 173.33 |
| 55 | $Ta_2O_5$ | 0.220447 | 94.07 |
| 56 | $SiO_2$ | 0.260142 | 161.27 |
| 57 | $Ta_2O_5$ | 0.239265 | 102.1 |
| 58 | $SiO_2$ | 0.323568 | 200.59 |
| 59 | $Ta_2O_5$ | 0.365131 | 155.81 |
| 60 | $SiO_2$ | 0.263255 | 163.2 |
| 61 | $Ta_2O_5$ | 0.047337 | 20.2 |
| 62 | $SiO_2$ | 0.047586 | 29.5 |
| 63 | $Ta_2O_5$ | 0.396322 | 169.12 |
| 64 | $SiO_2$ | 0.222976 | 138.23 |
| 65 | $Ta_2O_5$ | 0.297616 | 127 |
| 66 | $SiO_2$ | 0.218202 | 135.27 |
| 67 | $Ta_2O_5$ | 0.267362 | 114.09 |
| 68 | $SiO_2$ | 0.220266 | 136.55 |
| 69 | $Ta_2O_5$ | 0.364826 | 155.68 |
| 70 | $SiO_2$ | 0.041376 | 25.65 |

(continued)

| Layer number | Material | Optical thickness | Physical thickness (nm) |
|---|---|---|---|
| 71 | $Ta_2O_5$ | 0.026645 | 11.37 |
| 72 | $SiO_2$ | 0.318068 | 197.18 |
| 73 | $Ta_2O_5$ | 0.345985 | 147.64 |
| 74 | $SiO_2$ | 0.332585 | 206.18 |
| 75 | $Ta_2O_5$ | 0.280181 | 119.56 |
| 76 | $SiO_2$ | 0.270207 | 167.51 |
| 77 | $Ta_2O_5$ | 0.239804 | 102.33 |
| 78 | $SiO_2$ | 0.272208 | 168.75 |
| 79 | $Ta_2O_5$ | 0.426622 | 182.05 |
| 80 | $SiO_2$ | 0.042101 | 26.1 |
| 81 | $Ta_2O_5$ | 0.04094 | 17.47 |
| 82 | $SiO_2$ | 0.27032 | 167.58 |
| 83 | $Ta_2O_5$ | 0.263941 | 112.63 |
| 84 | $SiO_2$ | 0.103705 | 64.29 |

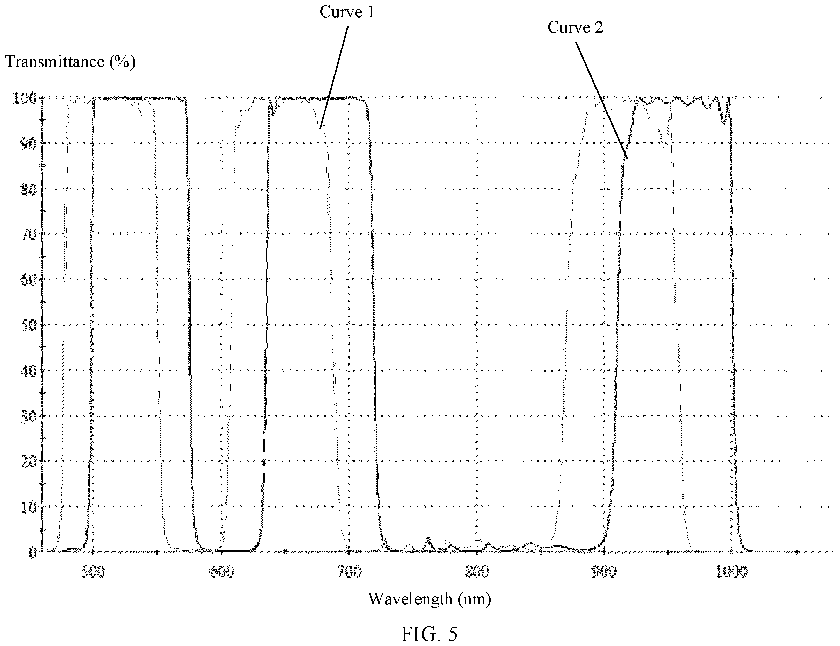

Further, refer to FIG. 5. FIG. 5 is a diagram of a transmittance curve of a thin-film optical filter according to an embodiment of this application. The transmittance curve is obtained through software optimization based on the target basic film Sub| $(1.5H0.7L)^3$ 2H $(0.7L1.5H)^3$ L $(1.5H0.7L)^3$ 2H $(0.7L1.5H)^3$|Air using $SiO_2$ as the substrate 21. An incident angle corresponding to a curve 1 is 30 degrees, and an incident angle corresponding to a curve 2 is 0 degrees. As shown in FIG. 5, an optimized bandpass filter 20 has triple bandpass performance.

In some feasible implementations, the bandpass filter 20 may alternatively be a quadruple bandpass filter, and an optical filter spectrum band of the bandpass filter 20 may specifically range 515 nm to 545 nm (which is assumed as a first optical filter spectrum band), 645 nm to 675 nm (which is assumed as a second optical filter spectrum band), 720 nm to 750 nm (which is assumed as a fourth optical filter spectrum band), and 925 nm to 955 nm (which is assumed as a third optical filter spectrum band). It should be understood that, in this case, there may also be four light source operating spectrum bands of the light source operating assembly 10, and the four light source operating spectrum bands may specifically include a first light source operating spectrum band, a second light source operating spectrum band, a third light source operating spectrum band, and a fourth light source operating spectrum band. The first optical filter spectrum band may include the first light source operating spectrum band, the second optical filter spectrum band may include the second light source operating spectrum band, the third optical filter spectrum band may include the third light source operating spectrum band, and the fourth optical filter spectrum band may include the fourth light source operating spectrum band.

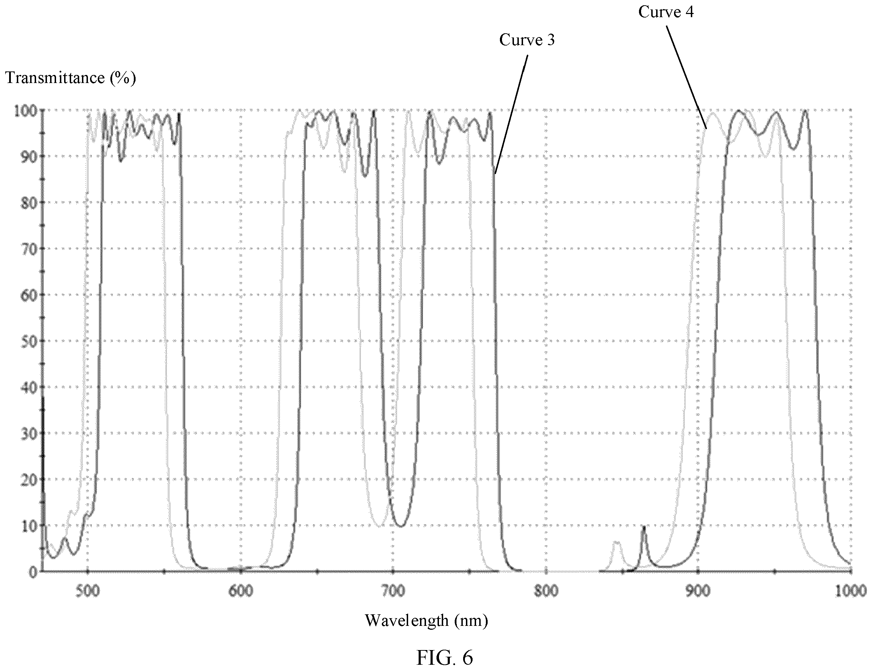

Further, refer to FIG. 6. FIG. 6 is a diagram of another transmittance curve of a thin-film optical filter according to an embodiment of this application. The transmittance curve is also obtained through software optimization based on the target basic film Sub| $(1.5H0.7L)^3$ 2H $(0.7L1.5H)^3$ L $(1.5H0.7L)^3$ 2H $(0.7L1.5H)^3$|Air using $SiO_2$ as the substrate 21. An incident angle corresponding to a curve 4 is 20 degrees, and an incident angle corresponding to a curve 5 is 0 degrees. As shown in FIG. 5, an optimized bandpass filter 20 has quadruple bandpass performance.

It can be learned from a plurality of tests that, the optical filter spectrum band of the bandpass filter 20 provided in this application has a wide coverage area, can cover visible light to a near infrared band, that is, 500 nm to 1000 nm, and has a large-angle characteristic. In a case of triple bandpass, an average transmittance of the three bands 515 nm to 545 nm, 645 nm to 675 nm, and 925 nm to 955 nm can be ensured to be greater than 92% in an incident angle range of 0° to 30°. In a case of quadruple bandpass, an average transmittance of the four bands 515 nm to 545 nm, 645 nm to 675 nm, 720 nm to 750 nm and 925 nm to 955 nm can be ensured to be greater than 90% in an incident angle range of 0° to 20°. In addition, the optical filter spectrum band of the bandpass filter 20 is wide, and a part whose transmittance is greater than 90% in each passband needs to exceed 30 nm. The bandpass filter 20 has a high transmittance. For the triple bandpass filter using $SiO_2$ as the substrate 21, when an incident angle is 0 degrees, an average transmittance in the first optical filter spectrum band 515 nm to 545 nm is greater than 92%, an average transmittance in the second optical filter spectrum band 645 nm to 675 nm is greater than 92%, and an average transmittance in the third optical filter spectrum band 925 nm to 955 nm is greater than 93%. When an incident angle is 30 degrees, an average transmittance in the first optical filter spectrum band 515 nm to 545 nm is greater than 91%, an average transmittance in the second optical filter spectrum band 645 nm to 675 nm is greater than 91%, and an average transmittance in the third optical filter spectrum band 925 nm to 955 nm is greater than 89%. The cut-off rate of the bandpass filter 20 is low, and an average transmittance in each suppression band (that is, a non-optical filter spectrum band) is less than 2%. Therefore, by using the bandpass filter 20 provided in this application, interference light can be effectively filtered out, and measurement accuracy of the optical measurement apparatus 100 is improved.

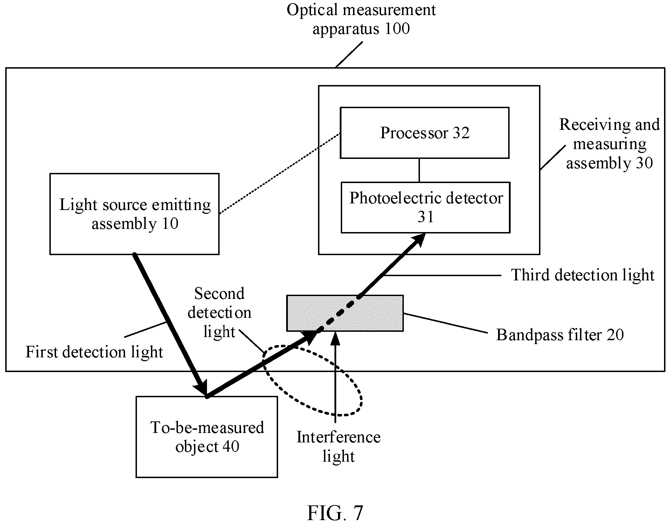

In some feasible implementations, refer to FIG. 7. FIG. 7 is a diagram of another structure of an optical measurement apparatus according to an embodiment of this application. As shown in FIG. 7, the receiving and measuring assembly 30 may specifically include a photoelectric detector 31 and a processor 32. The processor 32 is connected to the photoelectric detector 31, and the photoelectric detector 31 is disposed on a side that is of the receiving and measuring assembly 30 and that faces the bandpass filter 20.

In actual operation, after the light source emitting assembly 10 emits the first detection light, the photoelectric detector 31 is configured to receive the third detection light, determine an electrical signal (for ease of differentiation, a first electrical signal is used as a substitute for description below) based on the third detection light, and send the first electrical signal to the processor 32. After receiving the first electrical signal, the processor 32 may further obtain a second electrical signal. Herein, the second electrical signal is mainly used by the light source emitting assembly 10 to generate the first detection light. In other words, the light source emitting assembly 10 generates the first detection light under driving of the second electrical signal. The processor 32 is further configured to determine the to-be-measured feature value of the to-be-measured object 40 based on the first electrical signal and the second electrical signal.

Optionally, the processor 32 may be further connected to the light source emitting assembly 10. In actual operation, the processor 32 may be further configured to generate the second electrical signal, and send the second electrical signal to the light source emitting assembly 10. In other words, the processor 32 may alternatively be used as a driver or a controller of the light source emitting assembly 10, to control running of the light source emitting assembly 10.

Optionally, in some actual application scenarios, the optical measurement apparatus 100 may be specifically a photoplethysmography PPG sensor, the to-be-measured object 40 may be a human body tissue (like a wrist, an ankle, or a chest), and the to-be-measured feature value is a human biological parameter (like a human body pulse wave, blood oxygen saturation, or a heart rate). The first electrical signal may indicate parameters such as optical power and a wavelength of the third detection light, and the second electrical signal may indicate parameters such as optical power and a wavelength of the first detection light. For example, the optical measurement apparatus 100 may be a PPG sensor configured to measure a pulse wave of a human body, the to-be-measured object 40 may be a wrist of the human body, and the to-be-measured feature value is the pulse wave of the human body. In actual operation, the light source emitting assembly 10 may emit the first detection light under driving of the second electrical signal. Herein, the second electrical signal may indicate the optical power of the first detection light. Then, the photoelectric detector 31 in the receiving and measuring assembly 30 may determine, based on the third detection light received by the photoelectric detector 31, the first electrical signal that indicates the optical power of the third detection light. Then, the processor 32 in the receiving and measuring assembly 30 may obtain the optical power of the first detection light and the optical power of the third detection light based on the first electrical signal and the second electrical signal, and further determine the pulse wave of the human body based on the optical power of the first detection light and the optical power of the third detection light.

It should be additionally noted that the light source emitting assembly 10 in this application may be specifically a light emitting component with a fixed or adjustable wavelength in any form, like a light emitting diode (light emitting diode, LED) and a near-infrared light source. The photoelectric detector 31 in this application may be a component that can be configured to implement an optical-to-electrical conversion function in any form, for example, a photodiode (photodiode, PD), a phototransistor, or a photoresistor. The processor 32 in this application may be specifically a component having a data processing function in any form, for example, a CPU, a general-purpose processor, a DSP, an ASIC, an FPGA or another programmable logic device, a transistor logic component, a hardware component, or any combination thereof. Forms of the foregoing components or function modules are not specifically limited in this application.

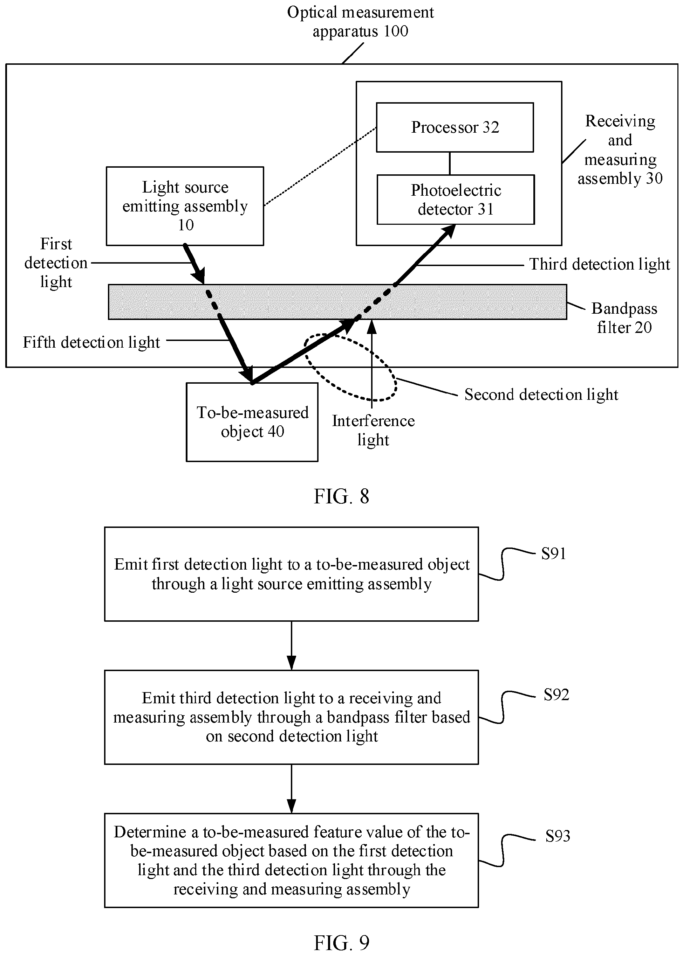

Herein, it should be further additionally noted that the foregoing structure (as shown in FIG. 1 or FIG. 7) of the optical measurement apparatus 100 is described by using an example in which the bandpass filter 20 is disposed only between the receiving and measuring assembly 30 and the to-be-measured object 40. In an optional implementation, refer to FIG. 8. FIG. 8 is a diagram of still another structure of an optical measurement apparatus according to an embodiment of this application. As shown in FIG. 8, the bandpass filter 20 may exist between the receiving and measuring assembly 30 and the to-be-measured object 40, and may exist between the light source emitting assembly 10 and the to-be-measured object 40. In other words, the bandpass filter 20 is large enough, so that both the first detection light emitted by the light source emitting assembly 10 and the second detection light emitted to the receiving and measuring assembly 30 pass through the bandpass filter 20. In actual operation, after the light source emitting assembly 10 emits the first detection light to the to-be-measured object 40, the bandpass filter 20 first receives the first detection light, and emits fifth detection light to the to-be-measured object 40. It should be understood that, because the optical filter spectrum band of the bandpass filter 20 includes the light source operating spectrum band of the light source emitting assembly 10, the fifth detection light is basically the same as the first detection light. The to-be-measured object 40 may reflect or transmit the fifth detection light.

Then, the bandpass filter 20 may emit third detection light to the receiving and measuring assembly 30 based on the second detection light. The second detection light may include light reflected or transmitted by the to-be-measured object 40 based on the fifth detection light. Then, the receiving and measuring assembly 30 may further determine and obtain the to-be-measured feature value of the to-be-measured object 40 based on the first detection light and the third detection light. By using such a structure, interference light can be effectively eliminated, and a problem that assembly is difficult because the bandpass filter 20 is disposed only on an incident light side of the receiving and measuring assembly 30 can be avoided, so that structure design and production of the optical measurement apparatus 100 are facilitated.

**Embodiment 2**

Refer to FIG. 9. FIG. 9 is a schematic flowchart of an optical measurement method according to an embodiment of this application. The optical measurement method is applicable to the optical measurement apparatus 100 described in Embodiment 1. In this embodiment, for a specific structure and a function of the optical measurement apparatus 100, refer to the corresponding descriptions in the foregoing Embodiment 1, and details are not described in this embodiment again. As shown in FIG. 9, the optical measurement method includes the following steps.

S91: Emit first detection light to a to-be-measured object through a light source emitting assembly.

In some feasible implementations, after the optical measurement apparatus 100 is normally started, the optical measurement apparatus 100 may first emit the first detection light to the to-be-measured object 40 through the light source emitting assembly 10. Specifically, after receiving a second electrical signal, the light source emitting assembly 10 may determine, based on the second electrical signal, optical power and a wavelength that are indicated by the second electrical signal, and emit the first detection light with the corresponding optical power and wavelength to the to-be-measured object. For a specific process, refer to the foregoing described process in which the light source emitting assembly 10 transmits the first detection light to the to-be-measured object 40. Details are not described herein again.

S92: Emit third detection light to a receiving and measuring assembly through a bandpass filter based on second detection light.

In some feasible implementations, after the first detection light is emitted to the to-be-measured object 40 through the light source emitting assembly 10, the optical measurement apparatus 100 may first perform, through the bandpass filter 20, optical filter on the second detection light received by the optical measurement apparatus 100, and emit the third detection light to the receiving and measuring assembly 30. Herein, the second detection light includes fourth detection light, that is, interference light, obtained by the to-be-measured object 40 by emitting or refracting the first detection light. The bandpass filter 20 is mainly configured to filter out the interference light, and transfer the fourth detection light to the receiving and measuring assembly 30 as completely as possible. Herein, for a specific process in which the optical measurement apparatus 100 emits the third detection light to the receiving and measuring assembly 30 based on the second detection light through the bandpass filter 20, refer to the foregoing description of the function of the bandpass filter 20. Details are not described herein again.

S93: Determine a to-be-measured feature value of the to-be-measured object based on the first detection light and the third detection light through the receiving and measuring assembly.

In some feasible implementations, the optical measurement apparatus 100 may determine the to-be-measured feature value of the to-be-measured object 40 based on the first detection light and the third detection light through the receiving and measuring assembly 30.

Specifically, the receiving and measuring assembly 30 may include a photoelectric detector 31 and a processor 32. The optical measurement apparatus 100 may determine a first electrical signal based on the third detection light through the photoelectric detector 31, and send the first electrical signal to the processor 32. Then, the optical measurement apparatus 100 determines the to-be-measured feature value based on the first electrical signal and the second electrical signal through the processor 32. The second electrical signal is mainly used by the light source emitting assembly 10 to generate the first detection light. For a specific process, refer to the description of the function of the receiving and measuring assembly 30 in Embodiment 1, and details are not described herein again.

In the optical measurement method provided in this application, the bandpass filter 20 disposed between the receiving and measuring assembly 30 and the to-be-measured object 40 may filter out interference light emitted to the receiving and measuring assembly 30, so that the receiving and measuring assembly 30 receives only a valid optical signal, and the interference signal is eliminated radically. Therefore, the optical measurement method provided in this application has high measurement accuracy and high practicability.

This application further provides a bandpass filter. For a structure of the bandpass filter, refer to FIG. 2. As shown in FIG. 2, the bandpass filter 20 mainly includes a substrate 21 and a thin-film optical filter 22 disposed on the substrate 21. Herein, for description of a structure and a function of the bandpass filter 20, refer to the descriptions of the structure and the function of the bandpass filter 20 in the foregoing Embodiment 1, and details are not described herein again.

The bandpass filter 20 provided in this application may have triple bandpass or quadruple bandpass performance, and an optical filter spectrum band of the bandpass filter has a wide coverage area, a large-angle characteristic, a large average transmittance of a passband, a low cut-off rate, and a small average transmittance of each suppression band (that is, a non-optical filter spectrum band). The bandpass filter 20 can effectively filter out light outside the optical filter spectrum band, and has high practicability.

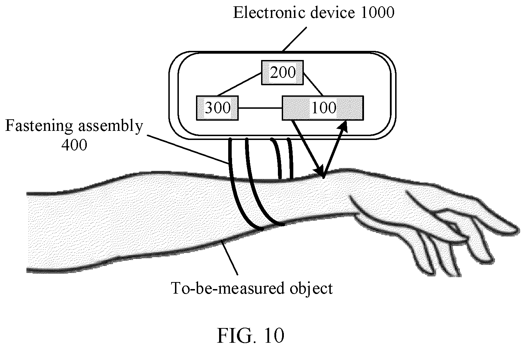

This application further provides an electronic device. Refer to FIG. 10. FIG. 10 is a diagram of a structure of an electronic device according to an embodiment of this application. As shown in FIG. 10, the electronic device 1000 may include the optical measurement apparatus 100 described above, a power supply 200, and a controller 300. The power supply 200, the optical measurement apparatus 100, and the controller 300 are coupled to each other. In actual operation, the power supply 200 is configured to supply power to the optical measurement apparatus 100 and the controller 300. The controller 300 is configured to control the optical measurement apparatus 100 to perform corresponding detection on a to-be-measured object 40, to obtain a corresponding to-be-measured feature value.

Optionally, as shown in FIG. 10, the electronic device 1000 may further include a fastening assembly 400. The fastening assembly 400 is mainly used for fastening between the electronic device 1000 and the to-be-measured object 40. For example, the fastening assembly 400 may be specifically a watchband, a waistband, or the like. The fastening assembly 400 ensures that the electronic device 1000 and the to-be-measured object 40 are fixed to each other, so that measurement accuracy of the optical measurement apparatus 100 can be improved.

Optionally, in actual application, the electronic device 1000 may be specifically a wearable device like a smartwatch (shown in a form of a smartwatch in FIG. 10), a portable heart rate meter, or a portable blood pressure meter. The optical measurement apparatus 100 is a PPG sensor included in the wearable device. The to-be-measured object 40 is a human body component. The to-be-measured feature value is a human biological parameter like a pulse wave, a heart rate, blood oxygen saturation, or blood pressure.

It should be noted herein that FIG. 10 shows only some function components included in the electronic device 1000. In an actual implementation, the electronic device 1000 may further include another component like a display and a microphone. A structure of the electronic device 1000 is not limited in this application.

It should be further noted herein that the controller 300 and the processor 32 may be a same component, or the processor 32 may be included in the controller 300. This is not specifically limited in this application.

In embodiments provided in this application, it should be understood that the disclosed system, apparatus, or method may be implemented in other manners. For example, the described apparatus embodiment is merely an example. For example, division into units is merely logical function division and may be other division in an actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the apparatuses or units may be implemented in electrical, mechanical, or other forms.

The units described as separate components may or may not be physically separate, and parts displayed as units may or may not be physical units, may be located in one place, or may be distributed on a plurality of network units. Some or all of the units may be selected based on actual requirements to achieve the objectives of the solutions of embodiments.

In addition, function units in embodiments of this application may be integrated into one processing unit, or each of the units may exist alone physically, or two or more units may be integrated into one unit.

In the specification, claims, and accompanying drawings of this application, the terms "first", "second", "third", "fourth" and so on are intended to distinguish between different objects but do not indicate a particular order. In addition, the terms "including" and "having" and any other variants thereof are intended to cover a non-exclusive inclusion. For example, a process, a method, a system, a product, or a device that includes a series of steps or units is not limited to the listed steps or units, but optionally further includes unlisted steps or units, or optionally further includes other inherent steps or units of the process, the method, the product, or the device.

An "embodiment" mentioned in the specification indicates that a particular feature, structure, or characteristic described with reference to this embodiment may be included in at least one embodiment of this application. The phrase shown in various locations in the specification may not necessarily refer to a same embodiment, and is not an independent or optional embodiment exclusive from another embodiment. It is explicitly and implicitly understood by a person skilled in the art that embodiments described in the specification may be combined with another embodiment.

A person skilled in the art should be aware that in the foregoing one or more examples, functions described in this application may be implemented by hardware, software, firmware, or any combination thereof. When the functions are implemented by software, the foregoing functions may be stored in a computer-readable medium or transmitted as one or more instructions or code in a computer-readable medium. The computer-readable medium includes a computer storage medium and a communication medium. The communication medium includes any medium that enables a computer program to be transmitted from one place to another place. The storage medium may be any available medium accessible to a general-purpose computer or a special-purpose computer.

In the foregoing specific implementations, the objectives, technical solutions, and beneficial effects of this application are further described in detail. It should be understood that the foregoing descriptions are merely specific implementations of this application, but are not intended to limit the protection scope of this application. Any modification, equivalent replacement, improvement, or the like made based on the technical solutions of this application shall fall within the protection scope of this application.

**Claims**

1. An optical measurement apparatus, wherein the optical measurement apparatus comprises a light source emitting assembly, a bandpass filter, and a receiving and measuring assembly, the bandpass filter is disposed between a to-be-measured object and the receiving and measuring assembly, and an optical filter spectrum band of the bandpass filter comprises a light source operating spectrum band of the light source emitting assembly, wherein

   the light source emitting assembly is configured to emit first detection light to the to-be-measured object;
   the bandpass filter is configured to emit third detection light to the receiving and measuring assembly based on second detection light, wherein the second detection light comprises light reflected or transmitted by the to-be-measured object based on the first detection light; and
   the receiving and measuring assembly is configured to determine a to-be-measured feature value of the to-be-measured object based on the first detection light and the third detection light.

2. The optical measurement apparatus according to claim 1, wherein the bandpass filter comprises a substrate and a thin-film optical filter disposed on the substrate, a film structure of the thin-film optical filter comprises at least two Fabry-Perot cavities disposed in series, structures of all of the at least two Fabry-Perot cavities are the same, a first Fabry-Perot cavity and a second Fabry-Perot cavity, which are any two adjacent cavities of the at least two Fabry-Perot cavities, are connected through a connection layer, and a side that is of the connection layer and that is connected to the first Fabry-Perot cavity and a side that is of the connection layer and that is connected to the second Fabry-Perot cavity each are a low refractive index layer of one basic thickness;

   a film structure of the first Fabry-Perot cavity is $(xHyL)^nkH(yLxH)^n$, wherein H represents a high refractive index layer of one basic thickness, L represents a low refractive index layer of one basic thickness, x is an optical thickness coefficient of the high refractive index layer, y is an optical thickness coefficient of the low refractive index layer, xH represents a high refractive index layer of x basic thicknesses, yL represents a low refractive index layer of y basic thicknesses, kH represents a high refractive index material layer of k basic thicknesses, n is a quantity of periodicities of a first membrane stack xHyL and a second membrane stack yLxH, and k is a multiple of 2; and
   the basic thickness is an optical thickness of a quarter of a center wavelength.

3. The optical measurement apparatus according to claim 2, wherein x is greater than or equal to 1 and less than or equal to 2, y is greater than or equal to 0.1 and less than or equal to 1, and n is greater than or equal to 1 and less than or equal to 10.

4. The optical measurement apparatus according to claim 2 or 3, wherein a material of the high refractive index layer comprises tantalum pentoxide $Ta_2O_5$, trititanium pentoxide $Ti_3O_5$, titanium dioxide $TaO_2$, or silicon nitride $Si_3N_4$.

5. The optical measurement apparatus according to any one of claims 2 to 4, wherein a material of the low refractive index layer comprises silicon dioxide $TiO_2$ or aluminum oxide $Al_2O_3$.

6. The optical measurement apparatus according to any one of claims 2 to 5, wherein a material of the substrate comprises silicon dioxide $SiO_2$, silicon Si, or transparent high polymer plastic.

7. The optical measurement apparatus according to any one of claims 2 to 6, wherein a center wavelength of the bandpass filter is greater than or equal to 880 nm and less than or equal to 920 nm.

8. The optical measurement apparatus according to any one of claims 1 to 7, wherein the receiving and measuring assembly comprises a photoelectric detection component and a processor, wherein

   the photoelectric detection component is configured to: determine a first electrical signal based on the third detection light, and send the first electrical signal to the processor; and
   the processor is configured to determine the to-be-measured feature value based on the first electrical signal and a second electrical signal, wherein the second electrical signal is used by the light source emitting assembly to generate the first detection light.

9. The optical measurement apparatus according to any one of claims 1 to 8, wherein the optical measurement apparatus is a photoplethysmography PPG sensor, the to-be-measured object is a human body tissue, and the to-be-measured feature value is a human biological parameter.

10. A bandpass filter, wherein the bandpass filter comprises a substrate and a thin-film optical filter disposed on the substrate, a film structure of the thin-film optical filter comprises at least two Fabry-Perot cavities disposed in series, structures all of the at least two Fabry-Perot cavities are the same, a first Fabry-Perot cavity and a second Fabry-Perot cavity, which are any two adjacent cavities of the at least two Fabry-Perot cavities, are connected through a connection layer, and a side that is of the connection layer and that is connected to the first Fabry-Perot cavity and a side that is of the connection layer and that is connected to the second Fabry-Perot cavity each are a low refractive index layer of one basic thickness;

a film structure of the first Fabry-Perot cavity is $(xHyL)^nkH(yLxH)^n$, wherein H represents a high refractive index layer of one basic thickness, L represents a low refractive index layer of one basic thickness, x is an optical thickness coefficient of the high refractive index layer, y is an optical thickness coefficient of the low refractive index layer, xH represents a high refractive index layer of x basic thicknesses, yL represents a low refractive index layer of y basic thicknesses, kH represents a high refractive index material layer of k basic thicknesses, n is a quantity of periodicities of a first membrane stack xHyL and a second membrane stack yLxH, and k is a multiple of 2; and
the basic thickness is an optical thickness of a quarter of a center wavelength.

11. The bandpass filter according to claim 10, wherein x is greater than or equal to 1 and less than or equal to 2, y is greater than or equal to 0.1 and less than or equal to 1, and n is greater than or equal to 1 and less than or equal to 10.

12. The bandpass filter according to claim 10 or 11, wherein a material of the high refractive index layer comprises tantalum pentoxide $Ta_2O_5$, trititanium pentoxide $Ti_3O_5$, titanium dioxide $TaO_2$, or silicon nitride $Si_3N_4$.

13. The bandpass filter according to any one of claims 10 to 12, wherein a material of the low refractive index layer comprises silicon dioxide $TiO_2$ or aluminum oxide $Al_2O_3$.

14. The bandpass filter according to any one of claims 10 to 13, wherein a material of the substrate comprises silicon dioxide $SiO_2$, silicon Si, or transparent high polymer plastic.

15. The bandpass filter according to any one of claims 10 to 14, wherein a center wavelength of the bandpass filter is greater than or equal to 880 nm and less than or equal to 920 nm.

16. An optical measurement method, wherein the method is applicable to an optical measurement apparatus, the optical measurement apparatus comprises a light source emitting assembly, a bandpass filter, and a receiving and measuring assembly, the bandpass filter is disposed between a to-be-measured object and the receiving and measuring assembly, and an optical filter spectrum band of the bandpass filter comprises a light source operating spectrum band of the light source emitting assembly; and
the method comprises:

emitting first detection light to the to-be-measured object through the light source emitting assembly;
emitting third detection light to the receiving and measuring assembly through the bandpass filter based on second detection light, wherein the second detection light comprises light reflected or transmitted by the to-be-measured object based on the first detection light; and
determining a to-be-measured feature value of the to-be-measured object based on the first detection light and the third detection light through the receiving and measuring assembly.

17. The method according to claim 16, wherein the receiving and measuring assembly comprises a photoelectric detector and a processor; and
the determining a to-be-measured feature value of the to-be-measured object based on the first detection light and the third detection light through the receiving and measuring assembly comprises:

determining a first electrical signal based on the third detection light through the photoelectric detector, and sending the first electrical signal to the processor; and

determining the to-be-measured feature value based on the first electrical signal and a second electrical signal through the processor, wherein the second electrical signal is used by the light source emitting assembly to generate the first detection light.

18. An electronic device, wherein the electronic device comprises the optical measurement apparatus according to any one of claims 1 to 9, a power supply, and a controller, and the optical measurement apparatus, the power supply, and the controller are coupled to each other, wherein

the power supply is configured to supply power to the optical measurement apparatus and the controller; and

the controller is configured to control the optical measurement apparatus to measure a to-be-measured object, to obtain a to-be-measured feature value of the to-be-measured object.

Optical measurement
apparatus 100

Light source emitting
assembly 10

Receiving and
measuring assembly
30

Third detection light

Second
detection
light

Bandpass filter 20

First detection light

To-be-measured
object 40

Interference
light

FIG. 1

Air 23

Bandpass filter 20

Thin-film
optical filter 22

Substrate 21

FIG. 2

▧ Low refractive index layer L
▨ High refractive index layer H

Another possible
Fabry-Perot cavity

Second
Fabry-Perot
cavity 222

Connection layer 223

$(yLxH)^n$

Second
membrane
stack 2212

$kH$

Spacer layer
2213

First
Fabry-Perot
cavity 221

$(xHyL)^n$

First
membrane
stack 2211

FIG. 3

Transmittance

Transmittance curve

Tmax

1/2Tmax

$2\Delta\lambda$

$\lambda 2$

Wavelength

FIG. 4

FIG. 5

FIG. 6

Optical measurement
apparatus 100

Processor 32

Receiving and
measuring
assembly 30

Light source emitting
assembly 10

Photoelectric detector
31

Third detection light

Second
detection
light

Bandpass filter 20

First detection light

To-be-measured
object 40

Interference
light

FIG. 7

Optical measurement
apparatus 100

Processor 32

Photoelectric
detector 31

Receiving
and
measuring
assembly 30

Light source
emitting assembly
10

First
detection
light

Third detection light

Bandpass
filter 20

Fifth detection light

Second detection light

To-be-measured
object 40

Interference
light

FIG. 8

Emit first detection light to a to-be-measured object
through a light source emitting assembly

S91

Emit third detection light to a receiving and
measuring assembly through a bandpass filter based
on second detection light

S92

Determine a to-be-measured feature value of the to-
be-measured object based on the first detection
light and the third detection light through the
receiving and measuring assembly

S93

FIG. 9

Electronic device 1000

200

Fastening assembly
400

300　　　100

To-be-measured object

FIG. 10

<table>
<tr><td colspan="2"><strong>INTERNATIONAL SEARCH REPORT</strong></td><td>International application No.<br><br>**PCT/CN2023/096493**</td></tr>
</table>

| | |
|---|---|
| **A.** | **CLASSIFICATION OF SUBJECT MATTER** |

G01N 21/31(2006.01)i;  G01N 21/25(2006.01)i

According to International Patent Classification (IPC) or to both national classification and IPC

| | |
|---|---|
| **B.** | **FIELDS SEARCHED** |

Minimum documentation searched (classification system followed by classification symbols)

IPC: G01N 21/-

Documentation searched other than minimum documentation to the extent that such documents are included in the fields searched

Electronic data base consulted during the international search (name of data base and, where practicable, search terms used)

CNKI, CNABS, CNTXT, ENTXTC, VEN, 光源, 发射, 接收, 带通滤光片, 反射, 透射, 法布里-珀罗, 法珀, 高折射率层, 低折射率层, 膜, light source, emit+, receiv+, band pass filter, reflect+, transmit+, Fabry Perot, high refractive index layer, low refractive index layer, film

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| X | CN 110763648 A (NANJING INSTITUTE OF ADVANCED LASER TECHNOLOGY) 07 February 2020 (2020-02-07)<br>    description, paragraphs 5-11 and figures 1-3 | 1, 16-18 |
| Y | CN 110763648 A (NANJING INSTITUTE OF ADVANCED LASER TECHNOLOGY) 07 February 2020 (2020-02-07)<br>    description, paragraphs 5-11 and figures 1-3 | 2-9 |
| X | CN 213843576 U (SUZHOU ZHONGWEI PHOTONICS CO., LTD.) 30 July 2021 (2021-07-30)<br>    description, paragraphs 6-15 and figure 1 | 10-15 |
| Y | CN 213843576 U (SUZHOU ZHONGWEI PHOTONICS CO., LTD.) 30 July 2021 (2021-07-30)<br>    description, paragraphs 6-15 and figure 1 | 2-9 |
| X | CN 104123539 A (CENTRAL SOUTH UNIVERSITY) 29 October 2014 (2014-10-29)<br>    description, paragraphs 37-53 and figure 1 | 1, 16-18 |

☑ Further documents are listed in the continuation of Box C.          ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **31 July 2023** | **01 August 2023** |

| Name and mailing address of the ISA/CN | Authorized officer |
|---|---|
| **China National Intellectual Property Administration (ISA/ CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/096493**

| C. | DOCUMENTS CONSIDERED TO BE RELEVANT | |
|---|---|---|
| Category* | Citation of document, with indication, where appropriate, of the relevant passages | Relevant to claim No. |
| Y | CN 104123539 A (CENTRAL SOUTH UNIVERSITY) 29 October 2014 (2014-10-29) description, paragraphs 37-53 and figure 1 | 2-9 |
| X | CN 103895915 A (SUZHOU JIANG'AO OPTOELECTRONICS TECHNOLOGY CO., LTD.) 02 July 2014 (2014-07-02) description, paragraphs 13-15 and figure 1 | 1, 16-18 |
| Y | CN 103895915 A (SUZHOU JIANG'AO OPTOELECTRONICS TECHNOLOGY CO., LTD.) 02 July 2014 (2014-07-02) description, paragraphs 13-15 and figure 1 | 2-9 |
| X | CN 112226729 A (SUZHOU ZHONGWEI PHOTONICS CO., LTD.) 15 January 2021 (2021-01-15) description, paragraphs 6-20 and figure 1 | 10-15 |
| Y | CN 112226729 A (SUZHOU ZHONGWEI PHOTONICS CO., LTD.) 15 January 2021 (2021-01-15) description, paragraphs 6-20 and figure 1 | 2-9 |
| A | CN 109932058 A (ZHEJIANG UNIVERSITY) 25 June 2019 (2019-06-25) entire document | 1-18 |
| A | US 5337191 A (PHOTRAN CORP.) 09 August 1994 (1994-08-09) entire document | 1-18 |
| A | WO 2022036511 A1 (SHENZHEN GOODIX TECHNOLOGY CO., LTD.) 24 February 2022 (2022-02-24) entire document | 1-18 |

Form PCT/ISA/210 (second sheet) (July 2022)

**INTERNATIONAL SEARCH REPORT**

International application No.

**PCT/CN2023/096493**

**Box No. III      Observations where unity of invention is lacking (Continuation of item 3 of first sheet)**

This International Searching Authority found multiple inventions in this international application, as follows:

The present application comprises two inventions, wherein the first invention comprises claims 1-9 and 16-18, and the second invention comprises claims 10-15. The common technical feature between independent claims 1, 16 and 18 in the first invention and independent claim 10 in the second invention merely lies in a bandpass filter. Since the common technical feature does not belong to a special technical feature that makes a contribution over the inventions, independent claims in the two inventions do not belong to a single general inventive concept, and do not meet the requirement of unity of invention (PCT Rule 13.1, 13.2 and 13.3).

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
**Information on patent family members**

International application No.

**PCT/CN2023/096493**

| Patent document cited in search report | | | Publication date (day/month/year) | Patent family member(s) | Publication date (day/month/year) |
|---|---|---|---|---|---|
| CN | 110763648 | A | 07 February 2020 | None | |
| CN | 213843576 | U | 30 July 2021 | None | |
| CN | 104123539 | A | 29 October 2014 | None | |
| CN | 103895915 | A | 02 July 2014 | None | |
| CN | 112226729 | A | 15 January 2021 | None | |
| CN | 109932058 | A | 25 June 2019 | None | |
| US | 5337191 | A | 09 August 1994 | None | |
| WO | 2022036511 | A1 | 24 February 2022 | None | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210622250